(19) Europäisches Patentamt
European Patent Office
Office européen des brevets

(11) Publication number: 0 136 745 B1

(12) # EUROPEAN PATENT SPECIFICATION

(45) Date of publication of patent specification: 07.06.89

(51) Int. Cl.$^4$: A 61 K 31/17

(21) Application number: 84201221.3

(22) Date of filing: 27.08.84

(54) **Benzoyl urea derivatives having anti-tumor activity.**

(30) Priority: 01.09.83 NL 8303043

(43) Date of publication of application: 10.04.85 Bulletin 85/15

(45) Publication of the grant of the patent: 07.06.89 Bulletin 89/23

(84) Designated Contracting States: AT BE CH DE FR GB IT LI LU NL SE

(56) References cited:
EP-A-0 025 363
EP-A-0 107 214

(73) Proprietor: DUPHAR INTERNATIONAL RESEARCH B.V
C.J. van Houtenlaan 36
NL-1381 CP Weesp (NL)

(72) Inventor: Brouwer, Marius S.
c/o OCTROOIBUREAU ZOAN B.V., Apollolaan 151
NL-1077 AR Amsterdam (NL)
Inventor: Van Hes, Roelof
c/o OCTROOIBUREAU ZOAN B.V., Apollolaan 151
NL-1077 AR Amsterdam (NL)

(74) Representative: Muis, Maarten et al
OCTROOIBUREAU ZOAN B.V. P.O. Box 140
NL-1380 AC Weesp (NL)

EP 0 136 745 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to new benzoyl urea derivatives having anti-tumor activity, to the preparation of such compounds, and to compositions comprising at least one of the new compounds as the active substance.

It is known from United States Patent Specification 3,748,356 that a group of 1-benzoyl-3-phenyl-urea compounds has insecticidal properties. Said insecticidal activity is based on a mechanism which has not yet been fully cleared. What is clear is that the insects are killed in that said urea derivatives inhibit the chitin formation.

Furthermore it is known from European Patent Application 0,025,363 that some N-benzoyl-N'-pyridyloxyphenyl urea compounds have an anti-tumor activity.

It has now been found that 1-benzoyl-3-phenyl-urea compounds containing at least one hydroxyl or mercapto group, or at least one group which can generate a hydroxyl or mercapto group have excellent anti-tumor activity. The new substituted benzoyl urea compounds according to the invention can be represented by the formula:

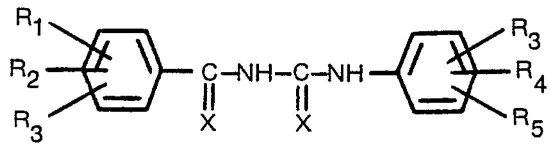

in which:

X is an oxygen or sulfur-atom;

$R_1$ is a hydrogen atom, a group (O—A)n, wherein A represents a hydrogen atom, an alkanoyl group having 1 to 6 carbon atoms, a carbamoyl group optionally substituted by alkyl containing 1 to 6 carbon atoms or by phenyl, a benzylcarbonyl group optionally substituted by halogen, a benzoyl group, a benzyl group, a phenyl group optionally substituted by halogen, or an alkoxycarbonylalkyl group containing from 1 to 6 carbon atoms, or $R_1$ is an alkylmercapto group, having 1 to 3 carbon atoms or a group —S—A, wherein A has the above given meaning;

$R_2$ is a hydrogen or halogen atom, an alkyl group having 1 to 6 carbon atoms optionally substituted by halogen, or $R_2$ is a nitro group;

$R_3$ is a hydrogen or halogen atom, a nitro group, a 1 to 6 carbon atoms containing alkyl group optionally substituted by halogen, an alkoxy group containing 1 to 6 carbon atoms optionally substituted by halogen, a cycloalkoxy group, containing 5—10 carbon atoms, optionally substituted by alkyl groups having 1—3 carbon atoms, an alkanoyl group containing from 1 to 6 carbon atoms, a benzoyl group, a nitrile group, an amino group optionally substituted by 1 or 2 alkyl groups having 1 to 3 carbon atoms, an alkoxycarbonyl group containing from 1 to 6 carbon atoms, or a piperidinocarbonyl group;

$R_4$ is a hydrogen atom, a group $(O—A)_m$ or S—A, wherein A has the above given meaning, or an alkylmercapto group wherein the alkyl group contains from 1 to 3 carbon atoms;

$R_5$ is a hydrogen atom, or represents from 1 to 3 halogen atoms;

n+m has the value from 1 to 6, with the proviso that $R_1$ and $R_4$ are not simultaneously hydrogen, and the following compounds are disclaimed:

1) 1-(2,6-difluorobenzoyl)-3-(2-hydroxy-4-chlorophenyl)urea
2) 1-(2,6-difluorobenzoyl)-3-(3-hydroxy-4-chlorophenyl)urea
3) 1-(2,6-difluorobenzoyl)-3-(4-hydroxyphenyl)urea
4) 1-(2,6-difluorobenzoyl)-3-(2-hydroxy-4-trifluoromethylphenyl)urea
5) 1-(2-chlorobenzoyl)-3-(2-hydroxy-4-trifluoromethoxyphenyl)urea
6) 1-(2-chlorobenzoyl)-3-(2-hydroxy-4-trifluoromethylphenyl)urea.

These disclaimed compounds are mentioned in the non-prepublished European patent application no. 83201263.7 (publication no. 0107214).

It has been found in particular that the compounds of the above formula wherein at least one of the phenyl rings contains a hydroxyl or an esterified hydroxyl group have a very strong anti-tumor activity in the test-model mentioned hereinafter.

The anti-tumor activity of the new compounds seems to be based on a new mechanism, which differs from that of known cytostatics. There are indications that the compounds according to the invention do not influence cell-cycle but are active on membrane-level. It has been found that the membrane-fluidity decreases under the influence of the new compounds.

The new compounds according to the invention can be obtained in a manner known for the synthesis of analogous compounds.

For example, the compounds according to the invention can be prepared by reacting a substituted aniline of the general formula

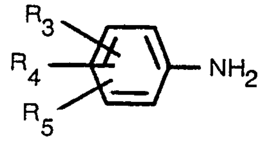

wherein $R_3$, $R_4$ and $R_5$ have the above given meanings, with a compound of the general formula

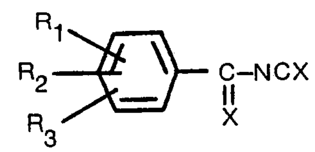

wherein $R_1$, $R_2$, $R_3$ and X have the above given meanings.

The new compounds according to the invention can also be prepared by reacting a substituted benzamide of the formula

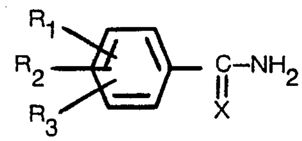

wherein $R_1$, $R_2$, $R_3$ and X have the above given meanings, with a compound of the formula

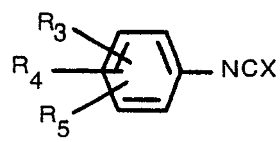

wherein $R_3$, $R_4$, $R_5$ and X have the above-mentioned meaning.

The above reactions are preferably carried out in the presence of an organic solvent, such as an aromatic hydrocarbon, an alkyl halide, a non-cyclic or cyclic dialkyl ether or acetonitrile, at a reaction temperature between 0°C and the boiling point of the solvent used.

Although the above mentioned methods of preparing are the best suitable, the new compounds can also be prepared in a different manner, for example as described in Netherlands patent application 7105350, or according to the methods described in Netherlands patent applications 7806678 or 8005588.

The compounds can be processed to compositions in any usual manner.

A great advantage of the compounds according to the invention as compared with known cytostatics is that they have a very low toxicity.

The anti-tumor activity was tested in an *in vitro* test. In this test the sensitivity of B16 melanoma cells to the new compounds according to the invention was determined.

B16 melanoma cells grow as a "monolayer". The doubling time of said cells in a cell culture is 12—16 hours. "6 Multi-well" tissue culture plates having a flat bottom having an area of 6 $cm^2$ proved to be best suited for carrying out the test.

On day 0 a quantity of $5 \times 10^4$ B16 melanoma cells is provided in each cell culture dish.

On day 1 the compounds to be tested are vortexed in the presence of glass-beads. The compound to be tested is then added to the dishes comprising melanoma cells in the desired quantity. Incubation takes place in a $CO_2$-incubator at 37°C for 20 hours. The incubation is terminated by removing the culture medium with therein the compound to be tested, after which the cells are washed twice and fresh medium is added.

Forty-eight hours after the beginning of the incubation the quantity of cells in each dish is measured by means of a microcell Coulter Counter. The results thus obtained are expressed by indicating the activity of the compounds as 1+, 2+, 3+, 4+ or 5+, which has the following meaning:

1+: cell growth is inhibited 1—20% with respect to control.
2+: cell growth in inhibited 20—40% with respect to control.
3+: cell growth is inhibited 40—60% with respect to control.
4+: cell growth is inhibited 60—80% with respect to control.
5+: cell growth is inhibited 80—100% with respect to control.

Sensitivity of B16 melanoma cells

Various compounds according to the invention were tested in the above-described manner in a dosage of 500 µg/ml in the form of an aqueous suspension. The results obtained are recorded in the table below:

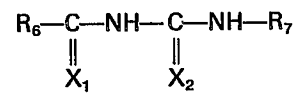

| Compound No. | $X_1$ | $X_2$ | $R_6$ | $R_7$ | M.P.(°C) | Activity |
|---|---|---|---|---|---|---|
| 1 | O | O | 2,6-dichloro-3-hydroxyphenyl | 4-chlorophenyl | 201 | 5+ |
| 2 | O | O | 2,6-dichloro-3-acetoxyphenyl | 4-chlorophenyl | 211 | 4+ |
| 3 | O | O | 2,6-dichloro-3-benzyloxyphenyl | 4-chlorophenyl | 151 | 3+ |
| 4 | O | O | 2,6-dichloro-3-hydroxyphenyl | 4-trifluoromethylphenyl | 204 | 5+ |
| 5 | O | O | 2,6-dichloro-3-acetoxyphenyl | 4-trifluoromethylphenyl | 169 | 5+ |
| 6 | O | O | 2-chloro-6-hydroxyphenyl | 4-chlorophenyl | 240 | 4+ |
| 7 | O | O | 2-chloro-6-acetoxyphenyl | 4-chlorophenyl | 174 | 5+ |
| 8 | O | O | 2-chloro-6-propionyloxyphenyl | 4-chlorophenyl | 159 | 4+ |
| 9 | O | O | 5-nitro-2-hydroxyphenyl | 4-chlorophenyl | 211 | 4+ |
| 10 | O | O | 2-hydroxyphenyl | 2-chloro-4-nitrophenyl | 221 | 4+ |
| 11 | O | O | 2,6-dihydroxyphenyl | 4-chlorophenyl | 280 | 5+ |
| 12 | O | O | 3,5-diiodo-2-hydroxyphenyl | 4-chlorophenyl | 187 | 5+ |
| 13 | O | O | 2,6-dihydroxyphenyl | 2-methyl-4-chlorophenyl | 201 | 4+ |
| 14 | O | O | 2-chloro-6-acetoxyphenyl | 4-(1,1,2,2-tetrafluoroethoxyphenyl | 105 | 5+ |
| 15 | O | O | 2-chloro-6-acetoxyphenyl | 4-methyloxyphenyl | 172 | 4+ |
| 16 | O | O | 2-chloro-6-acetoxyphenyl | 4-piperidinocarbonylphenyl | 172 | 5+ |
| 17 | O | O | 2-benzoyloxyphenyl | 4-chlorophenyl | 183 | 4+ |
| 18 | O | O | 3,5-dichloro-4-hydroxyphenyl | 4-chlorophenyl | 244 | 4+ |
| 19 | O | O | 3,5-dichloro-4-acetoxyphenyl | 4-chlorophenyl | 127 | 4+ |

| Compound No. | $X_1$ | $X_2$ | $R_6$ | $R_7$ | M.P.(°C) | Activity |
|---|---|---|---|---|---|---|
| 20 | O | O | 5-chloro-2-hydroxyphenyl | 2-chloro-4-nitrophenyl | 228 | 3+ |
| 21 | O | O | 2,6-dichloro-4-hydroxyphenyl | 3,4-dichlorophenyl | 260 | 4+ |
| 22 | O | O | 2,6-dibenzyloxyphenyl | 2-methyl-4-chlorophenyl | 206 | 3+ |
| 23 | O | O | 2-chloro-6-acetoxyphenyl | 4-benzoylphenyl | 155 | 5+ |
| 24 | O | O | 2,6-difluorophenyl | 4-chloro-2-acetoxyphenyl | 150 | 5+ |
| 25 | O | O | 2,6-difluorophenyl | 4-trifluoromethyl-2-acetoxyphenyl | 162 | 5+ |
| 26 | O | O | 2,6-difluorophenyl | 3-trifluoromethyl-4-hydroxyphenyl | 194 | 5+ |
| 27 | O | O | 2-chlorophenyl | 3-trifluoromethyl-4-hydroxyphenyl | 209 | 3+ |
| 28 | O | O | 2-chlorophenyl | 2-trifluoromethoxy-4-hydroxyphenyl | 156 | 5+ |
| 29 | O | O | 2-chlorophenyl | 4-trifluoromethyl-2-acetoxyphenyl | 178 | 3+ |
| 30 | O | O | 2-chlorophenyl | 4-trifluoromethoxy-2-acetoxyphenyl | 170 | 3+ |
| 31 | O | O | 2,6-difluorophenyl | 3-chloro-4-(4-chlorophenoxy)phenyl | 189 | 3+ |
| 32 | O | O | 2,6-dichlorophenyl | 2-methylcarbamoyloxyphenyl | 186 | 4+ |
| 33 | O | O | 2,6-dichlorophenyl | 3-phenylcarbamoyloxyphenyl | 166 | 5+ |
| 34 | O | O | 2-chlorophenyl | 3-chloro-4-(4-chlorobenzylcarbonyloxy)phenyl | 184 | 3+ |
| 35 | O | O | 2,6-difluorophenyl | 3-trifluoromethyl-4-ethoxycarbonylmethoxyphenyl | 128 | 4+ |
| 36 | O | O | 2-methylphenyl | 4-hydroxyphenyl | 214 | 4+ |
| 37 | O | O | 2-chlorophenyl | 3,5-di-tert. butyl-4-hydroxyphenyl | 80 | 5+ |
| 38 | O | O | 2-chloro-6-hydroxyphenyl | 4-chloro-3-hydroxyphenyl | 198 | 4+ |
| 39 | O | O | 2-chloro-6-acetoxyphenyl | 4-chloro-3-acetoxyphenyl | 181 | 5+ |
| 40 | O | O | 2-chloro-6-acetoxyphenyl | 4-hydroxyphenyl | 140 | 4+ |

| Compound No. | $X_1$ | $X_2$ | $R_6$ | $R_7$ | M.P.(°C) | Activity |
|---|---|---|---|---|---|---|
| 41 | O | O | 2-chloro-6-acetoxyphenyl | 5-methyl-2-hydroxyphenyl | 185 | 5+ |
| 42 | O | O | 2-chloro-6-acetoxyphenyl | 4-nitro-2-hydroxyphenyl | 181 | 5+ |
| 43 | O | O | 2-chloro-6-acetoxyphenyl | 5-methyl-2-acetoxyphenyl | 190 | 5+ |
| 44 | O | O | 2-chloro-6-acetoxyphenyl | 4-n-butoxycarbonyl-3-hydroxyphenyl | 159 | 4+ |
| 45 | O | O | 2-chloro-6-acetoxyphenyl | 3,5-dibromo-2-hydroxyphenyl | 185 | 5+ |
| 46 | O | O | 2-chloro-6-acetoxyphenyl | 3-acetyl-4-hydroxyphenyl | 181 | 5+ |
| 47 | O | O | 3,5-di-tert. butyl-4-hydroxyphenyl | 4-acetoxyphenyl | 205 | 4+ |
| 48 | O | O | 3-chloro-4-acetoxyphenyl | 4-hydroxyphenyl | 250 | 4+ |
| 49 | O | O | 2-acetoxyphenyl | 3,5-dichloro-4-hydroxyphenyl | 201 | 5+ |
| 50 | O | O | 2,6-difluorophenyl | 2-mercaptophenyl | 187 | 5+ |
| 51 | O | S | phenyl | 2-acetoxyphenyl | 138 | 4+ |
| 52 | S | S | 2-chloro-6-methylthiophenyl | 4-chlorophenyl | 170 | 5+ |
| 53 | O | S | 2,4-dichlorophenyl | 3-hydroxyphenyl | 155 | 5+ |
| 54 | O | O | 2-benzyloxyphenyl | 4-chlorophenyl | 185 | 2+ |
| 55 | O | O | 2,6-difluorophenyl | 3,5-dichloro-4-(4-chlorobenzoyl)phenyl | 237 | 3+ |
| 56 | O | O | 3-chloro-4-acetoxyphenyl | 3-trifluoromethyl-4-hydroxyphenyl | 225 | 3+ |
| 57 | O | O | 2,6-difluorophenyl | 4-(4-chlorophenoxycarbonyloxy)phenyl | 220 | 2+ |

The anti-tumor activity of the compounds is confirmed by preliminary results obtained in an *in vivo* test in mice.

The preparation of the compounds is illustrated in the following examples:

Example 1

To a stirred solution of 2.67 g of 2-hydroxy-3,5-dibromoaniline in 5 ml. of dry ether was added slowly 2.39 g of 2-acetoxy-6-chlorobenzoylisocyanate. After stirring overnight the precipitate was collected and washed with ether. Yield: 1.88 g of 1-(2-acetoxy-6-chlorobenzoyl)-3-(2-hydroxy-3,5-dibromophenyl)urea (compound no. 45 as listed above) having a melting point of 185°C.

The acetoxy group of the obtained compound was removed by boiling the compound in a solution of ethanol/HCl (2N) for a few hours. In this manner the corresponding compound carrying a free hydroxyl group is obtained.

Example II

A solution of 4.5 g of 2-benzyloxybenzamide and 3.07 g of 4-chlorophenylisocyanate in 50 ml of dry xylene was refluxed during 16 hours. After cooling the precipitate was collected and washed with ether. Compound no. 54, i.e. 1-(2-benzyloxybenzoyl)-3-(4-chlorophenyl)urea was obtained in a yield of 6.9 g; m.p. 185°C.

The benzyl group was removed by heating the obtained compound in acetic acid/HBr at 55—60°C for a few hours.

The above listed compounds all have been prepared according to the methods of the described examples.

**Claims for the Contracting States: BE CH DE FR GB IT LI LU NL SE**

1. Compounds having anti-tumour activity of the formula

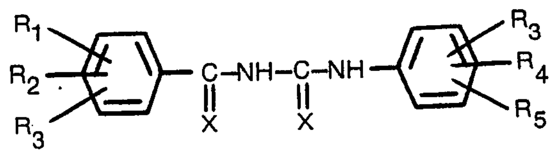

wherein

X is an oxygen or sulfur-atom;

$R_1$ is a hydrogen atom, a group $(O—A)_n$, wherein A represents a hydrogen atom, an alkanoyl group having 1 to 6 carbon atoms, a carbamoyl group optionally substituted by alkyl containing 1 to 6 carbon atoms or by phenyl, a benzylcarbonyl group optionally substituted by halogen, a benzoyl group, a benzyl group, a phenyl group optionally substituted by halogen, or an alkoxycarbonylalkyl group containing from 1 to 6 carbon atoms, or $R_1$ is an alkylmercapto group, having 1 to 3 carbon atoms or a group —S—A, wherein A has the above given meaning;

$R_2$ is a hydrogen or halogen atom, an alkyl group having 1 to 6 carbon atoms optionally substituted by halogen, or $R_2$ is a nitro group;

$R_3$ is a hydrogen or halogen atom, a nitro group, a 1 to 6 carbon atoms containing alkyl group optionally substituted by halogen, an alkoxy group containing 1 to 6 carbon atoms optionally substituted by halogen, a cycloalkoxy group, containing 5—10 carbon atoms, optionally substituted by alkyl groups having 1—3 carbon atoms, an alkanoyl group containing from 1 to 6 carbon atoms, a benzoyl group, a nitrile group, an amino group optionally substituted by 1 or 2 alkyl groups having 1 to 3 carbon atoms, an alkoxycarbonyl group containing from 1 to 6 carbon atoms, or a piperidinocarbonyl group;

$R_4$ is a hydrogen atom, a group $(O—A)_m$ or S—A, wherein A has the above given meaning, or an alkylmercapto group wherein the alkyl group contains from 1 to 3 carbon atoms;

$R_5$ is a hydrogen atom, or represents from 1 to 3 halogen atoms;

n+m has the value from 1 to 6, with the proviso that $R_1$ and $R_4$ are not simultaneously hydrogen, and the following compounds are disclaimed:

1) 1-(2,6-difluorobenzoyl)-3-(2-hydroxy-4-chlorophenyl)urea;
2) 1-(2,6-difluorobenzoyl)-3-(3-hydroxy-4-chlorophenyl)urea;
3) 1-(2,6-difluorobenzoyl)-3-(4-hydroxyphenyl)urea;
4) 1-(2,6-difluorobenzoyl)-3-(2-hydroxy-4-trifluoromethylphenyl)urea;
5) 1-(2-chlorobenzoyl)-3-(2-hydroxy-4-trifluoromethoxyphenyl)urea;
6) 1-(2-chlorobenzoyl)-3-(2-hydroxy-4-trifluoromethylphenyl)urea.

2. Compounds according to claim 1, wherein $R_2$, $R_3$, $R_5$ and X have the meanings given in claim 1 and $R_1$ and $R_4$ represent from 1 to 6 hydroxyl groups or esterified hydroxyl groups.

3. Compounds according to claim 2, wherein esterified hydroxyl groups are acetoxy groups.

4. A composition having anti-tumour activity, characterized in that it contains at least one compound according to claim 1 as active component.

5. A method of preparing compounds as claimed in claim 1, characterized in that a) a compound of the formula

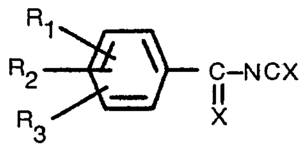

is reacted with a compound of the formula

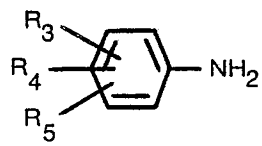

or b) a compound of the formula

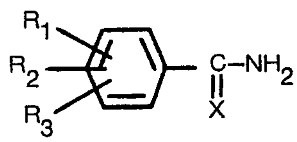

is reacted with a compound of the formula

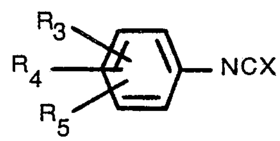

in which formulae $R_1$ to $R_5$ and X have the meanings given in claim 1.

6. Use of a compound as claimed in claim 1 for the manufacture of a composition having anti-tumour activity.

**Claims for the Contracting State: AT**

1. A method of preparing benzoyl urea compounds, characterized in that compounds of the formula

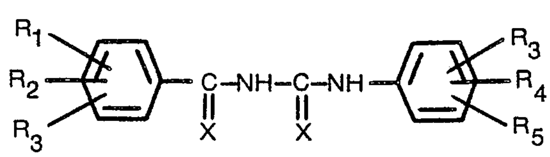

wherein

X is an oxygen or sulfur-atom;

$R_1$ is a hydrogen atom, a group $(O—A)_n$, wherein A represents a hydrogen atom, an alkanoyl group having 1 to 6 carbon atoms, a carbamoyl group optionally substituted by alkyl containing 1 to 6 carbon atoms or by phenyl, a benzylcarbonyl group optionally substituted by halogen, a benzoyl group, a benzyl group, a phenyl group optionally substituted by halogen, or an alkoxycarbonylalkyl group containing from 1 to 6 carbon atoms, or $R_1$ is an alkylmercapto group, having 1 to 3 carbon atoms or a group —S—A, wherein A has the above given meaning;

$R_2$ is a hydrogen or halogen atom, an alkyl group having 1 to 6 carbon atoms optionally substituted by halogen, or $R_2$ is a nitro group;

$R_3$ is a hydrogen or halogen atom, a nitro group, a 1 to 6 carbon atoms containing alkyl group optionally substituted by halogen, an alkoxy group containing 1 to 6 carbon atoms optionally substituted by halogen, a cycloalkoxy group, containing 5—10 carbon atoms, optionally substituted by alkyl groups having 1—3 carbon atoms, an alkanoyl group containing from 1 to 6 carbon atoms, a benzoyl group, a nitrile group, an amino group optionally substituted by 1 or 2 alkyl groups having 1 to 3 carbon atoms, an alkoxycarbonyl group containing from 1 to 6 carbon atoms, or a piperidinocarbonyl group;

$R_4$ is a hydrogen atom, a group $(O—A)_m$ or S—A, wherein A has the above meaning, or an alkylmercapto group wherein the alkyl group contains from 1 to 3 carbon atoms;

$R_5$ is a hydrogen atom, or represents from 1 to 3 halogen atoms;

n+m has the value from 1 to 6, with the proviso that $R_1$ and $R_4$ are not simultaneously hydrogen, and the following compounds are disclaimed:

1) 1-(2,6-difluorobenzoyl)-3-(2-hydroxy-4-chlorophenyl)urea;
2) 1-(2,6-difluorobenzoyl)-3-(3-hydroxy-4-chlorophenyl)urea;
3) 1-(2,6-difluorobenzoyl)-3-(4-hydroxyphenyl)urea;
4) 1-(2,6-difluorobenzoyl)-3-(2-hydroxy-4-trifluoromethylphenyl)urea;
5) 1-(2-chlorobenzoyl)-3-(2-hydroxy-4-trifluoromethoxyphenyl)urea;
6) 1-(2-chlorobenzoyl)-3-(2-hydroxy-4-trifluoromethylphenyl)urea;

are prepared by reacting

a) a compound of the formula

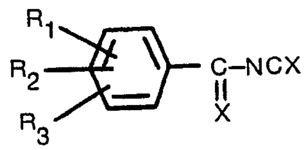

with a compound of the formula

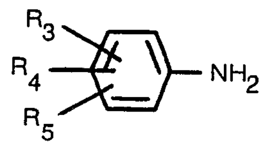

or b) by reacting a compound of the formula

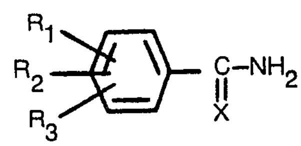

with a compound of the formula

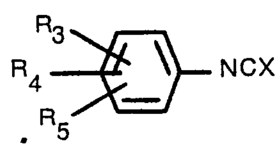

2. Use of a compound as claimed in claim 1 for the manufacture of a composition having anti-tumour activity.

**Patentansprüche für die Vertragsstaaten: BE CH DE FR GB IT LI LU NL SE**

1. Verbindungen mit Antitumorwirksamkeit der Formel

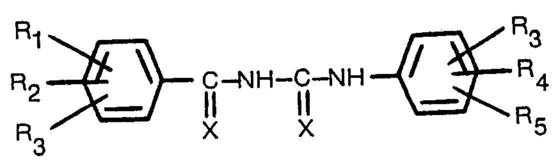

worin X Sauerstoff- oder Schwefelatom ist; $R_1$ ein Waserstoffatom, eine Gruppe $(O—A)_n$, wobei A ein Wasserstoffatom, eine Alkanoylgruppe mit 1 bis 6 Kohlenstoffatomen, eine Carbamoylgruppe gegebenenfalls substituiert durch Alkyl mit 1 bis 6 Kohlenstoffatomen oder durch Phenyl, eine Benzylcarbonylgruppe gegebenenfalls substituiert durch Halogen, eine Benzoylgruppe, eine Benzylgruppe, eine Phenylgruppe gegebenenfalls substituiert durch Halogen, oder eine Alkoxycarbonylalkylgruppe mit 1 bis 6 Kohlenstoffatomen bedeutet, oder $R_1$ eine Alkylmercaptogruppe mit 1 bis 3 Kohlenstoffatomen oder eine Gruppe —S—A, wobei A die oben angegebene Bedeutung hat, darstellt; $R_2$ ein Wasserstoff- oder Halogenatom, eine Alkylgruppe mit 1 bis 6 Kohlenstoffatomen gegebenenfalls substituiert durch Halogen oder $R_2$ eine Nitrogruppe ist; $R_3$ eine Wasserstoff- oder Halogenatom, eine Nitrogruppe, eine 1 bis 6 Kohlenstoffatome enthaltende Alkylgruppe gegebenenfalls substituiert durch Halogen, eine Alkoxygruppe mit 1 bis 6 Kohlenstoffatomen gegebenenfalls substituiert durch Halogen, eine Cycloalkoxygruppe mit 5 bis 10 Kohlenstoffatomen gegebenenfalls substituiert durch Alkylgruppen mit 1 bis 3 Kohlenstoffatomen, eine Alkanoylgruppe mit 1 bis 6 Kohlenstoffatomen, eine Benzoylgruppe, eine Nitrilgruppe, eine Aminogruppe gegebenenfalls substituiert durch 1 oder 2 Alkylgruppen mit 1 bis 3 Kohlenstoffatomen, eine Alkoxycarbonylgruppe mit 1 bis 6 Kohlenstoffatomen oder eine Piperidinocarbonylgruppe darstellt; $R_4$ ein Wasserstoffatom, eine Gruppe $(O—A)_m$ oder S—A,

wobei A die oben angegebene Bedeutung hat, oder eine Alkylmercaptogruppe, in der die Alkylgruppe 1 bis 3 Kohlenstoffatome aufweist, ist; $R_5$ ein Wasserstoffatom bedeutet oder 1 bis 3 Halogenatome darstellt und n + m den Wert 1 bis 6 haben, mit der Maßgabe, daß $R_1$ und $R_4$ nicht gleichzeitig Wasserstoff sind und die folgenden Verbindungen ausgenommen sind:

1) 1-(2,6-Difluorbenzoyl)-3-(2-hydroxy-4-chlorphenyl)-harnstoff,
2) 1-(2,6-Difluorbenzoyl)-3-(3-hydroxy-4-chlorphenyl)-harnstoff,
3) 1-(2,6-Difluorbenzoyl)-3-(4-hydroxyphenyl)-harnstoff,
4) 1-(2,6-Difluorbenzoyl)-3-(2-hydroxy-4-trifluormethylphenyl)harnstoff,
5) 1-(2-Chlorbenzoyl)-3-(2-hydroxy-4-trifluormethoxyphenyl)-harnstoff,
6) 1-(2-Chlorbenzoyl)-3-(2-hydroxy-4-trifluormethylphenyl)-harnstoff.

2. Verbindung nach Anspruch 1, worin $R_2$, $R_3$, $R_5$ und X die in Anspruch 1 angegebenen Bedeutungen haben und $R_1$ und $R_4$ 1 bis 6 Hydroxylgruppen oder veresterte Hydroxylgruppen darstellen.

3. Verbindungen nach Anspruch 2, worin veresterte Hydroxylgruppen Acetoxygruppen sind.

4. Zusammensetzung mit Antitumorwirksamkeit, dadurch gekennzeichnet, daß sie mindestens eine Verbindung nach Anspruch 1 als aktiven Bestandteil enthält.

5. Verfahren zum Herstellen von Verbindungen, wie in Anspruch 1 beansprucht, dadurch gekennzeichnet, daß

a) eine Verbindung der Formel

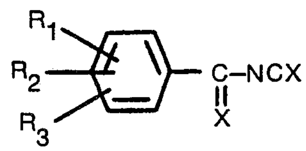

mit einer Verbindung der Formel

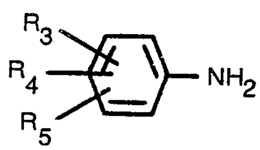

umgesetzt wird oder

b) eine Verbindung der Formel

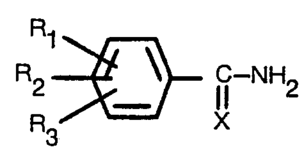

mit einer Verbindung der Formel

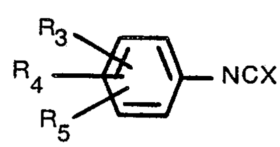

umgesetzt wird, in welchen Formeln $R_1$ bis $R_5$ und X die in Anspruch 1 angegebenen Bedeutungen haben.

6. Verwendung einer Verbindung, wie in Anspruch 1 beansprucht, zur Herstellung einer Zusammensetzung mit Antitumorwirksamkeit.

**Patentansprüche für den Vertragsstaat: AT**

1. Verfahren zum Herstellen von Benzoylharnstoffverbindungen, dadurch gekennzeichnet, daß Verbindungen der Formel

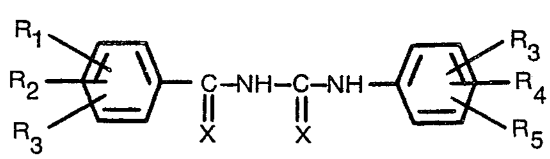

worin X ein Sauerstoff- oder Schwefelatom ist; $R_1$ ein Waserstoffatom, eine Gruppe $(O—A)_n$, wobei A ein Wasserstoffatom, eine Alkanoylgruppe mit 1 bis 6 Kohlenstoffatomen, eine Carbamoylgruppe

gegebenenfalls substituiert durch Alkyl mit 1 bis 6 Kohlenstoffatomen oder durch Phenyl, eine Benzylcarbonylgruppe gegebenenfalls substituiert durch Halogen, eine Benzoylgruppe, eine Benzylgruppe, eine Phenylgruppe gegebenenfalls substituiert durch Halogen, oder eine Alkoxycarbonylalkylgruppe mit 1 bis 6 Kohlenstoffatomen bedeutet, oder $R_1$ eine Alkylmercaptogruppe mit 1 bis 3 Kohlenstoffatomen oder eine Gruppe —S—A, wobei A die oben angegebene Bedeutung hat, darstellt; $R_2$ ein Wasserstoff- oder Halogenatom, eine Alkylgruppe mit 1 bis 6 Kohlenstoffatomen gegebenenfalls substituiert durch Halogen oder $R_2$ eine Nitrogruppe ist; $R_3$ eine Wasserstoff- oder Halogenatom, eine Nitrogruppe, eine 1 bis 6 Kohlenstoffatome enthaltende Alkylgruppe gegebenenfalls substituiert durch Halogen, eine Alkoxygruppe mit 1 bis 6 Kohlenstoffatomen gegebenenfalls substituiert durch Halogen, eine Cycloalkoxygruppe mit 5 bis 10 Kohlenstoffatomen gegebenenfalls substituiert durch Alkylgruppen mit 1 bis 3 Kohlenstoffatomen, eine Alkanoylgruppe mit 1 bis 6 Kohlenstoffatomen, eine Benzoylgruppe, eine Nitrilgruppe, eine Aminogruppe gegebenenfalls substituiert durch 1 oder 2 Alkylgruppen mit 1 bis 3 Kohlenstoffatomen, eine Alkoxycarbonylgruppe mit 1 bis 6 Kohlenstoffatomen oder eine Piperidinocarbonylgruppe darstellt; $R_4$ ein Wasserstoffatom, eine Gruppe $(O—A)_m$ oder S—A, wobei A die oben angegebene Bedeutung hat, oder eine Alkylmercaptogruppe, in der die Alkylgruppe 1 bis 3 Kohlenstoffatome aufweist, ist; $R_5$ ein Wasserstoffatom bedeutet oder 1 bis 3 Halogenatome darstellt und n + m den Wert 1 bis 6 haben, mit der Maßgabe, daß $R_1$ und $R_4$ nicht gleichzeitig Wasserstoff sind und die folgenden Verbindungen ausgenommen sind:

1) 1-(2,6-Difluorbenzoyl)-3-(2-hydroxy-4-chlorphenyl)-harnstoff,
2) 1-(2,6-Difluorbenzoyl)-3-(3-hydroxy-4-chlorphenyl)-harnstoff,
3) 1-(2,6-Difluorbenzoyl)-3-(4-hydroxyphenyl)-harnstoff,
4) 1-(2,6-Difluorbenzoyl)-3-(2-hydroxy-4-trifluormethylphenyl)harnstoff,
5) 1-(2-Chlorbenzoyl)-3-(2-hydroxy-4-trifluormethoxyphenyl)-harnstoff,
6) 1-(2-Chlorbenzoyl)-3-(2-hydroxy-4-trifluormethylphenyl)-harnstoff,

hergestellt werden durch Umsetzen

a) eine Verbindung der Formel

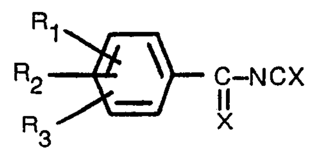

mit einer Verbindung der Formel

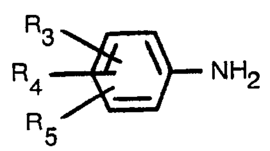

oder

b) einer Verbindung der Formel

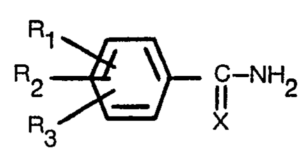

mit einer Verbindung der Formel

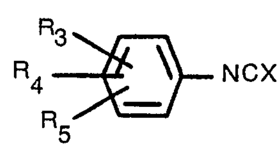

2. Verwendung einer Verbindung, wie in Anspruch 1 beansprucht, zur Herstellung einer Zusammensetzung mit Antitumorwirksamkeit.

**Revendications pour les Etats contractants: BE CH DE FR GB IT LI LU NL SE**

1. Composés ayant une activité antitumorale, de formule

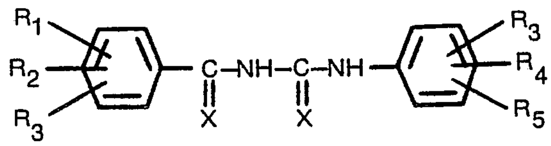

dans laquelle

X est un atome d'oxygène ou de soufre;

$R_1$ est un atome d'hydrogène, un groupe $(O{-}A)_n$, dans lequel A représente un atome d'hydrogène, un groupe alcanoyle comportant 1 à 6 atomes de carbone, un groupe carbamoyle éventuellement substitué par un groupe alkyle contenant 1 à 6 atomes de carbone ou par un groupe phényle, un groupe benzylcarbonyle éventuellement substitué par un halogène, un groupe benzoyle, un groupe benzyle, un groupe phényle éventuellement substitué par un halogène, ou un groupe alcoxycarbonylalkyle contenant de 1 à 6 atomes de carbone, ou $R_1$ est un groupe alkylmercapto comportant 1 à 3 atomes de carbone ou un groupe —S—A dans lequel A a la signification indiquée ci-dessus;

$R_2$ est un atome d'hydrogène ou d'halogène, un groupe alkyle comportant 1 à 6 atomes de carbone, éventuellement substitué par un halogène, ou $R_2$ est un groupe nitro;

$R_3$ est un atome d'hydrogène ou d'halogène, un groupe nitro, un groupe alkyle contenant 1 à 6 atomes de carbone éventuellement substitué par un halogène, un groupe alcoxy contenant 1 à 6 atomes de carbone éventuellement substitué par un halogène, un groupe cycloalcoxy contenant 5—10 atomes de carbone, éventuellement substitué par des groupes alkyle comportant 1—3 atomes de carbone, un groupe alcanoyle contenant de 1 à 6 atomes de carbone, un groupe benzoyle, un groupe nitrile, un groupe amino éventuellement substitué par un ou deux groupes alkyle comportant 1 à 3 atomes de carbone, un groupe alcoxycarbonyle contenant de 1 à 6 atomes de carbone, ou un groupe pipéridinocarbonyle;

$R_4$ est un atome d'hydrogène, un groupe $(O{-}A)_m$ ou S—A, dans lequel A a la signification indiqué ci-dessus, ou un groupe alkylmercapto dans lequel le groupe alkyle contient de 1 à 3 atomes de carbone;

$R_5$ est un atome d'hydrogène ou représente de 1 à 3 atomes d'halogène;

n+m a la valeur de 1 à 6, à condition que $R_1$ et $R_4$ ne soient pas simultanément un hydrogène, les composés suivants n'étant pas revendiqués:

1) la 1-(2,6-difluorobenzoyl)-3-(2-hydroxy-4-chlorophényl)urée;
2) la 1-(2,6-difluorobenzoyl)-3-(3-hydroxy-4-chlorophényl)urée;
3) la 1-(2,6-difluorobenzoyl)-3-(4-hydroxyphényl)urée;
4) la 1-(2,6-difluorobenzoyl)-3-(2-hydroxy-4-trifluorométhylphényl)urée;
5) la 1-(2,6-difluorobenzoyl)-3-(2-hydroxy-4-trifluorométhoxyphényl)urée;
6) la 1-(2,6-difluorobenzoyl)-3-(2-hydroxy-4-trifluorométhylphényl)urée.

2. Composés selon la revendication 1, dans lesquels $R_2$, $R_3$, $R_5$ et X ont les significations indiquées dans la revendication 1, et $R_1$ et $R_4$ représentent de 1 à 6 groupes hydroxyle ou groupes hydroxyle estérifiés.

3. Composés selon la revendication 2, dans lesquels les groupes hydroxyle estérifiés sont des groupes acétoxy.

4. Composition ayant une activité antitumorale, caractérisée en ce qu'elle contient au moins un composé selon la revendication 1 comme constituant actif.

5. Procédé de préparation des composés selon la revendication 1, caractérisé en ce que 1) on fait réagir un composé de formule

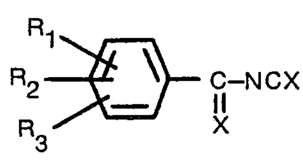

avec un composé de formule

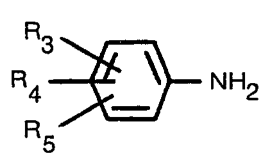

ou b) on fait réagir un composé de formule

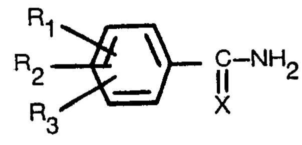

avec un composé de formule

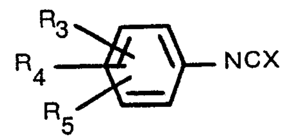

formules dans lesquelles $R_1$ à $R_5$ et X ont les significations indiquées dans la revendication 1.

6. Utilisation d'un composé selon la revendication 1 pour fabriquer une composition ayant une activité antitumorale.

**Revendications pour l'Etat contractant: AT**

1. Procédé de préparation de composés benzoylurées, caractérisé en ce que l'on prépare des composés de formule

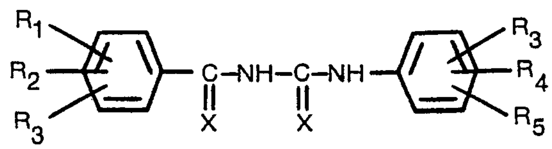

dans laquelle

X est un atome d'oxygène ou de soufre;

$R_1$ est un atome d'hydrogène, un groupe $(O—A)_n$, dans lequel A représente un atome d'hydrogène, un groupe alcanoyle comportant 1 à 6 atomes de carbone, un groupe carbamoyle éventuellement substitué par un groupe alkyle contenant 1 à 6 atomes de carbone ou par un groupe phényle, un groupe benzylcarbonyle éventuellement substitué par un halogène, un groupe benzoyle, un groupe benzyle, un groupe phényle éventuellement substitué par un halogène, ou un groupe alcoxycarbonylalkyle contenant de 1 à 6 atomes de carbone, ou $R_1$ est un groupe alkylmercapto comportant 1 à 3 atomes de carbone ou un groupe —S—A dans lequel A a la signification indiquée ci-dessus;

$R_2$ est un atome d'hydrogène ou d'halogène, un groupe alkyle comportant 1 à 6 atomes de carbone, éventuellement substitué par un halogène, ou $R_2$ est un groupe nitro;

$R_3$ est un atome d'hydrogène ou d'halogène, un groupe nitro, un groupe alkyle contenant 1 à 6 atomes de carbone éventuellement substitué par un halogène, un groupe alcoxy contenant 1 à 6 atomes de carbone éventuellement substitué par un halogène, un groupe cycloalcoxy contenant 5—10 atomes de carbone, éventuellement substitué par des groupes alkyle comportant 1—3 atomes de carbone, un groupe alcanoyle contenant de 1 à 6 atomes de carbone, un groupe benzoyle, un groupe nitrile, un groupe amino éventuellement substitué par un ou deux groupes alkyle comportant 1 à 3 atomes de carbone, un groupe alcoxycarbonyle contenant de 1 à 6 atomes de carbone, ou un groupe pipéridinocarbonyle;

$R_4$ est un atome d'hydrogène, un groupe $(O—A)_m$ ou S—A, dans lequel A a la signification indiqué ci-dessus, ou un groupe alkylmercapto dans lequel le groupe alkyle contient de 1 à 3 atomes de carbone;

$R_5$ est un atome d'hydrogène ou représente de 1 à 3 atomes d'halogène;

n+m a la valeur de 1 à 6, à condition que $R_1$ et $R_4$ ne soient pas simultanément un hydrogène, les composé suivants n'étant pas revendiqués:

1) la 1-(2,6-difluorobenzoyl)-3-(2-hydroxy-4-chlorophényl)urée;
2) la 1-(2,6-difluorobenzoyl)-3-(3-hydroxy-4-chlorophényl)urée;
3) la 1-(2,6-difluorobenzoyl)-3-(4-hydroxyphényl)urée;
4) la 1-(2,6-difluorobenzoyl)-3-(2-hydroxy-4-trifluorométhylphényl)urée;
5) la 1-(2-chlorobenzoyl)-3-(2-hydroxy-4-trifluorométhoxyphényl)urée;
6) la 1-(2-chlorobenzoyl)-3-(2-hydroxy-4-trifluorométhylphényl)urée.

en faisant réagir

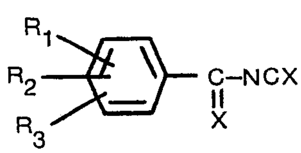

avec un composé de formule

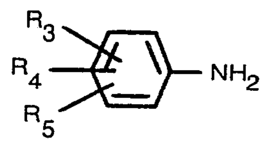

ou b) on faisant réagir un composé de formule

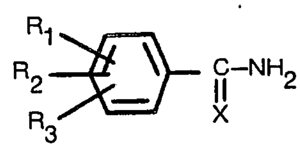

avec un composé de formule

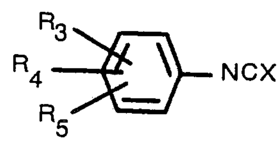

2. Utilisation d'un composé selon la revendication 1 pour fabriquer une composition ayant un activité antitumorale.